# EUROPEAN PATENT SPECIFICATION

(11) **EP 3 109 185 B1**
(45) Date of publication and mention of the grant of the patent: **07.02.2018**
(21) Application number: 16173752.3
(22) Date of filing: 09.06.2016
(51) Int. Cl.: B65F 1/06, G01N 33/00

(54) **METHOD AND DEVICE FOR PROMPTING CHANGE OF GARBAGE BAG**
VERFAHREN UND VORRICHTUNG ZUR AUFFORDERUNG ZUM WECHSELN DES MÜLLBEUTELS
PROCÉDÉ ET DISPOSITIF D'INCITATION À CHANGER UN SAC À ORDURES MÉNAGÈRES

(30) Priority: 26.06.2015 CN 201510364123
(43) Date of publication of application: 28.12.2016
(73) Proprietor: Xiaomi Inc., Beijing 100085 (CN)
(72) Inventor: WU, Ke, 100085 BEIJING (CN); LIU, Xinyu, 100085 BEIJING (CN)
(74) Representative: Delumeau, François Guy

(56) References cited:
- WO-A1-2013/163362
- CN-A- 104 648 861
- US-A1- 2009 161 907
- US-B1- 6 425 487

## Description

### TECHNICAL FIELD

The present invention generally relates to the field of communication technology, and more particularly, to method and device for prompting change of garbage bag.

### BACKGROUND

With the rapid development of social economics and the quick improvement of people's life standards, great amounts of garbage have been generated in daily production and life. People typically keep garbage in a garbage can, into which a garbage bag is placed when the garbage can is in use, and when there is too much garbage in the garbage bag, the bag will be taken out and dropped.

When a lot of garbage has been placed into a garbage can, since people often forget to drop a garbage bag, mold or odor will be produced due to the garbage's spoiling after accumulated for a long time and, which not only significantly affects human health but also pollutes the environment, and needs to be addressed urgently.

Document CN 104648861 discloses a method according to the preamble of claim 1 and a device according to the preamble of claim 10 by disclosing a smart bucket, which includes a smell sensor, a controller and an announciator.

### SUMMARY

Embodiments of the present invention provide a method and a device for prompting change of garbage bag, serving to change a garbage bag in which mold or odor has been produced.

In a first aspect of embodiments, the invention relates to a method for prompting change of garbage bag, the method comprising: acquiring garbage information and garbage spoiling feature information in a current garbage bag; determining whether garbage in the current garbage bag has produced mold or odor based on the garbage information and the garbage spoiling feature information; and when it is determined that the garbage has produced mold or odor, sending a user prompt information for changing the garbage bag. According to the invention, the step of acquiring garbage spoiling feature information comprises: acquiring a garbage type in the current garbage bag; and acquiring garbage spoiling feature information corresponding to the garbage type.

According to the invention is the method for prompting the change of a garbage bag performed by a mobile terminal.

In an exemplary embodiment, the step of acquiring a garbage type in the current garbage bag comprises: acquiring the garbage type by scanning a barcode on a packing box or on a packing bag of the garbage; or acquiring the garbage type by identifying the garbage via a camera; or acquiring the garbage type by making a spectroscopic analysis on composition of the garbage; or receiving the garbage type reported by a garbage can.

In an exemplary embodiment, the step of acquiring garbage spoiling feature information corresponding to the garbage type comprises: querying a preset database based on the garbage type to acquire garbage spoiling feature information corresponding to the garbage type, the preset database storing spoiling feature information of different garbage types; or acquiring garbage spoiling feature information corresponding to the garbage type from prestored history record information; or acquiring garbage spoiling feature information corresponding to the garbage type based on user's review information.

In an exemplary embodiment, the step of acquiring garbage information in the current garbage bag comprises: acquiring garbage image information in the current garbage bag via a camera; and/or acquiring garbage smell information in the current garbage bag via a smell sensor.

In an exemplary embodiment, the step of determining whether garbage in the current garbage bag has produced mold or odor based on the garbage information and the garbage spoiling feature information comprises: determining whether the garbage information is consistent with at least a part of the garbage spoiling feature information, the garbage spoiling feature information being used to determine whether the garbage in the current garbage bag has produced mold or odor.

In an exemplary embodiment, the method further comprises: before sending the user the prompt information for changing the garbage bag, acquiring behavior information of the user and a distance between the user and a garbage can; and determining whether the distance is smaller than a preset threshold and whether the behavior information is in a preset list, the preset list being used to store information of a behavior that can produce odor.

In an exemplary embodiment, the step of sending a user prompt information for changing the garbage bag comprises: when it is determined that the distance is smaller than the preset threshold and the behavior information is not in the preset list, sending the user the prompt information for changing the garbage bag.

In an exemplary embodiment, the step of sending a user prompt information for changing the garbage bag comprises: displaying the prompt information for changing the garbage bag via a mobile terminal; and/or prompting the user to read the prompt information for changing the garbage bag on the mobile terminal via a wearable device; and/or when it is detected that the mobile terminal is in the same local area network as a garbage can, sending the garbage can a first instruction, which is used to control the garbage can to make a sound to prompt the user to change the garbage bag; and/or when it is detected that the mobile terminal is in the same local area network as the garbage can, displaying the prompt information for changing the garbage bag via a terminal device which includes a TV.

In an exemplary embodiment, the method further comprises: after sending the user the prompt information for changing the garbage bag, receiving garbage-bag-unchanged prompt information sent by a garbage can and sending a smart device a second instruction used to control the smart device to seal the garbage bag or drop the current garbage bag.

In a second aspect of embodiments, the invention relates to a device for prompting change of garbage bag, the device comprising: an acquiring module configured to acquire garbage information and garbage spoiling feature information in a current garbage bag; a first determining module configured to determine whether garbage in the current garbage bag has produced mold or odor based on the garbage information and the garbage spoiling feature information acquired by the acquiring module; and a prompt information sending module configured to, when the first determining module determines that the garbage has produced mold or odor, send a user prompt information for changing the garbage bag. According to the invention, the acquiring module comprises: a type acquiring sub-module configured to acquire a garbage type in the current garbage bag; and garbage spoiling feature information acquiring sub-module configured to acquire garbage spoiling feature information corresponding to the garbage type.

According to the invention is the device for prompting the change of a garbage bag in a mobile terminal.

In an exemplary embodiment, the type acquiring sub-module comprises: a scanning unit configured to acquire the garbage type by scanning a barcode on a packing box or on a packing bag of the garbage; or an identifying unit configured to acquire the garbage type by identifying the garbage via a camera; or a spectroscopic analyzing unit configured to acquire the garbage type by making a spectroscopic analysis on composition of the garbage; or a type receiving unit configured to receive the garbage type reported by a garbage can.

In an exemplary embodiment, the garbage spoiling feature information acquiring sub-module comprises: a first acquiring unit configured to query a preset database based on the garbage type to acquire garbage spoiling feature information corresponding to the garbage type, the preset database storing spoiling feature information of different garbage types; or a second acquiring unit configured to acquire garbage spoiling feature information corresponding to the garbage type from prestored history record information; or a third acquiring unit configured to acquire garbage spoiling feature information corresponding to the garbage type based on user's review information.

In an exemplary embodiment, the acquiring module comprises: an image acquiring sub-module configured to acquire garbage image information in the current garbage bag via a camera; and/or a smell acquiring sub-module configured to acquire garbage smell information in the current garbage bag via a smell sensor.

In an exemplary embodiment, the first determining module is configured to: determine whether the garbage information is consistent with at least a part of the garbage spoiling feature information, the garbage spoiling feature information being used to determine whether the garbage in the current garbage bag has produced mold or odor.

In an exemplary embodiment, the device further comprises: an information acquiring module configured to, before the prompt information sending module sends the user the prompt information for changing the garbage bag, acquire behavior information of the user and a distance between the user and a garbage can; and a second determining module configured to determine whether the distance is smaller than a preset threshold and whether the behavior information is in a preset list, the preset list being used to store information of a behavior that can produce odor.

In an exemplary embodiment, the prompt information sending module is configured to: when it is determined that the distance is smaller than the preset threshold and the behavior information is not in the preset list, send the user the prompt information for changing the garbage bag.

In an exemplary embodiment, the prompt information sending module comprises: a first displaying sub-module configured to display the prompt information for changing the garbage bag via a mobile terminal; and/or a read prompting sub-module configured to prompt the user to read the prompt information for changing the garbage bag on the mobile terminal via a wearable device; and/or an instruction sending sub-module configured to, when it is detected that the mobile terminal is in the same local area network as a garbage can, send the garbage can a first instruction, which is used to control the garbage can to make a sound to prompt the user to change the garbage bag; and/or a second displaying sub-module configured to, when it is detected that the mobile terminal is in the same local area network as the garbage can, display the prompt information for changing the garbage bag via a terminal device which includes a TV.

In an exemplary embodiment, the device further comprises: a receiving and controlling module configured to, after the prompt information sending module sends the user the prompt information for changing the garbage bag, receive garbage-bag-unchanged prompt information sent by a garbage can and send a smart device a second instruction used to control the smart device to seal the garbage bag or drop the current garbage bag. Embodiments relate also to a device for prompting change of garbage bag, the device comprising: a processor; and a memory storing instructions executable by the processor; the processor is configured to: acquire garbage information and garbage spoiling feature information in a current garbage bag; determine whether garbage in the current garbage bag has produced mold or odor based on the garbage information and the garbage spoiling feature information; and when it is determined that the garbage has produced mold or odor, send a user prompt information for changing the garbage bag.

In one particular embodiment, the steps of the above method for prompting change of garbage bag are determined by computer program instructions. Embodiments are also directed to a computer program for executing the steps of a method for prompting change of garbage bag as described above when this program is executed by a computer.

This program can use any programming language and take the form of source code, object code or a code intermediate between source code and object code, such as a partially compiled form, or any other desirable form. Embodiments are also directed to a computer-readable information medium containing instructions of a computer program as described above.

The information medium can be any entity or device capable of storing the program. For example, the information medium can include storage means such as a ROM, for example a CD ROM or a microelectronic circuit ROM, or magnetic storage means, for example a diskette (floppy disk) or a hard disk.

Alternatively, the information medium can be an integrated circuit in which the program is incorporated, the circuit being adapted to execute the method in question or to be used in its execution.

The technical solutions provided by embodiments of the present invention may include the following advantageous effects: by acquiring garbage information and garbage spoiling feature information in a current garbage bag, determining whether garbage in the current garbage bag has produced mold or odor based on the garbage information and the garbage spoiling feature information, and sending a user prompt information for changing the garbage bag when it is determined that the garbage has produced mold or odor to enable the user to change the garbage bag, the impact on human health and the pollution of surrounding environment by garbage can be reduced effectively and use experience of the user can be improved since the embodiments prompt user to change the current garbage bag when it is determined that garbage has produced mold or odor.

It is to be understood that both the forgoing general description and the following detailed description are exemplary only, and are not restrictive of the present invention.

### BRIEF DESCRIPTION OF THE DRAWINGS

The accompanying drawings, which are incorporated in and constitute a part of this specification, illustrate embodiments consistent with the invention and, together with the description, serve to explain the principles of the invention.
Fig. 1 is a flow chart showing a method for prompting change of garbage bag according to an exemplary embodiment.
Fig. 2 is a scenario diagram showing a method for prompting change of garbage bag according to an exemplary embodiment.
Fig. 3 is a flow chart showing another method for prompting change of garbage bag according to an exemplary embodiment.
Fig. 4 is a flow chart showing a further method for prompting change of garbage bag according to an exemplary embodiment.
Fig. 5 is a block diagram showing a device for prompting change of garbage bag according to an exemplary embodiment.
Fig. 6A is a block diagram showing another device for prompting change of garbage bag according to an exemplary embodiment.
Fig. 6B is a block diagram showing another device for prompting change of garbage bag according to an exemplary embodiment.
Fig. 7 is a block diagram showing another device for prompting change of garbage bag according to an exemplary embodiment.
Fig. 8 is a block diagram showing another device for prompting change of garbage bag according to an exemplary embodiment.
Fig. 9 is a block diagram showing another device for prompting change of garbage bag according to an exemplary embodiment.
Fig. 10 is a block diagram applicable to a device for prompting change of garbage bag according to an exemplary embodiment.

### DETAILED DESCRIPTION

Reference will now be made in detail to exemplary embodiments, examples of which are illustrated in the accompanying drawings. The following description refers to the accompanying drawings in which same numbers in different drawings represent same or similar elements unless otherwise described. The implementations set forth in the following description of exemplary embodiments do not represent all implementations consistent with the invention. Instead, they are merely examples of apparatuses and methods consistent with aspects related to the invention as recited in the appended claims.

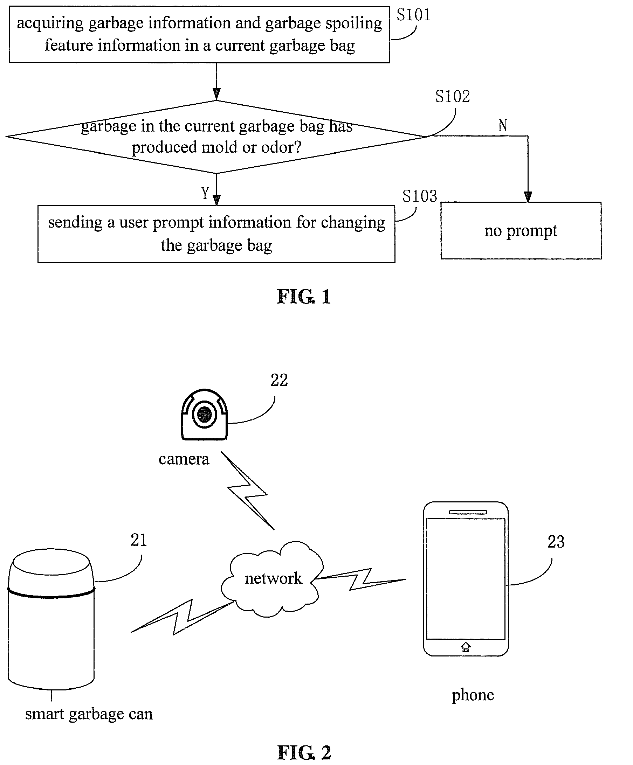

Fig. 1 is a flow chart showing a method for prompting change of garbage bag according to an exemplary embodiment. As shown in Fig. 1, the method for prompting change of garbage bag is performed by a mobile terminal, which may be a terminal device capable of accessing the Internet such as a phone, a PAD, etc. The method may include the following steps S101-S103.

In step S101, garbage information and garbage spoiling feature information in a current garbage bag may be acquired.

In the embodiment, a mobile terminal such as a phone may acquire a garbage type in the current garbage bag and acquire garbage spoiling feature information corresponding to the garbage type.

The mobile terminal such as a phone may acquire the garbage type in the current garbage bag in, but not limited to, the following ways.

The first way is by scanning a barcode on a packing box or on a packing bag of the garbage.

The second way is by identifying the garbage via a camera.

The third way is by making a spectroscopic analysis on composition of the garbage.

The fourth way is by receiving the garbage type reported by a garbage can, wherein the garbage type reported by the garbage can may be acquired by the garbage can in the first to third ways as above.

The mobile terminal such as a phone may acquire garbage spoiling feature information corresponding to the garbage type in, but not limited to, the following ways: it may query a preset database based on the garbage type to acquire garbage spoiling feature information corresponding to the garbage type, the preset database storing spoiling feature information of different garbage types; it may also acquire garbage spoiling feature information from prestored history record information; it may also acquire garbage spoiling feature information corresponding to the garbage type based on user's review information.

As can be seen, the present embodiment may acquire garbage spoiling feature information in flexible and diverse ways.

Meanwhile, garbage image information in the current garbage bag may be acquired via a camera, and garbage smell information in the current garbage bag may be acquired via a smell sensor.

As important to note, the garbage can mentioned in the present embodiment is a smart garbage can with communication functions.

In step S102, it may be determined whether garbage in the current garbage bag has produced mold or odor based on the garbage information and the garbage spoiling feature information.

In the embodiment, it may be determined whether garbage in the current garbage bag has produced mold or odor by determining whether the garbage information is consistent with at least a part of the garbage spoiling feature information, wherein the garbage spoiling feature information is used to determine whether the garbage in the current garbage bag has produced mold or odor.

For example, picture information acquired may be compared with picture information of garbage which has produced mold; and if their matching degree is higher than a preset value, it is determined that the garbage in the current garbage bag has produced mold.

In step S103, when it is determined that the garbage in the current garbage bag has produced mold or odor, prompt information for changing the garbage bag may be sent to a user.

The mobile terminal may directly send the prompt information for changing the garbage bag to the user when it is determined that the garbage in the current garbage bag has produced mold or odor, and may not send the prompt information when it is determined that the garbage in the current garbage bag has not produced mold or odor.

However, in some cases such as when the user is going to a toilet, the smell sensor may detect that a garbage can beside the toilet has odor and report the odor information to the mobile terminal; at this time, the mobile terminal will prompt the user to change the garbage bag due to misjudgments. In order to avoid such a situation, before sending the prompt information for changing the garbage bag to the user, the method may further include: acquiring behavior information of the user and the distance between the user and the garbage can; determining whether the distance is smaller than a preset threshold and whether the behavior information is in a preset list; when it is determined that the distance is smaller than the preset threshold and the behavior information is in the preset list, not sending the user the prompt information for changing the garbage bag; sending the user the prompt information for changing the garbage bag in all other cases, wherein the preset list is used to store information of a behavior that can produce odor. Behavior information of the user may be collected by a wearable device such as bracelet, and distance information may be acquired based on Bluetooth signal intensity information.

As can be seen, the above way can effectively reduce situations in which prompt information is sent to a user due to misjudgments, thus effectively reduces interruption to users and improves the degree of use experience of the user.

In the present embodiment, the mobile terminal may send the user the prompt information for changing the garbage bag in the following one or more ways: it may directly display the prompt information for changing the garbage bag via the mobile terminal; it may also prompt the user to read the prompt information for changing the garbage bag on the mobile terminal via a wearable device such as a bracelet; it may also send the garbage can a first instruction when it is detected that the user has come back home, which instruction is used to control the garbage can to send the prompt information for changing the garbage bag; that is, the garbage can makes a prompt sound after receiving the first instruction; it may also display the prompt information for changing the garbage bag via a terminal device such as a TV when it is detected that the user has come back home. As can be seen, the present embodiment may send the user the prompt information for changing the garbage bag in flexible and diverse ways.

The way to detect that the user has come back home may be by detecting that the mobile terminal is in the same local area network as the garbage can, or by detecting that the distance with the garbage can obtained via a Bluetooth signal intensity detection is within a preset range, etc.

Below, the present invention is illustrated with reference to Fig. 2. As shown in Fig. 2, when a user is using a smart garbage can 21, the user may make a spectroscopic analysis on composition of the garbage to be placed into the smart garbage can 21 via a spectrometer in the smart garbage can 21 so as to acquire a garbage type (assumed as type A), and query a database such as a database of an online shopping site based on garbage of type A to acquire garbage spoiling feature information of the garbage of type A. Then, garbage image information in the current garbage bag may be acquired via a camera 22, and the garbage image information and corresponding garbage spoiling feature information may be compared by a phone 23; if it is found that the matching degree between the garbage image information and image information of garbage which has produced mold is higher than 90%, prompt information for changing the garbage bag may be displayed on the phone 23, upon seeing which, the user will change the garbage bag timely, so that pollution of surrounding environment is reduced and use experience of the user is improved.

With the above embodiment of the method for prompting change of garbage bag, by acquiring garbage information and garbage spoiling feature information in a current garbage bag, determining whether garbage in the current garbage bag has produced mold or odor based on the garbage information and the garbage spoiling feature information, and sending a user prompt information for changing the garbage bag when it is determined that the garbage has produced mold or odor to enable the user to change the garbage bag, the impact on human health and the pollution of surrounding environment by garbage can be reduced effectively and use experience of the user can be improved since the embodiment prompts user to change the current garbage bag when it is determined that garbage has produced mold or odor.

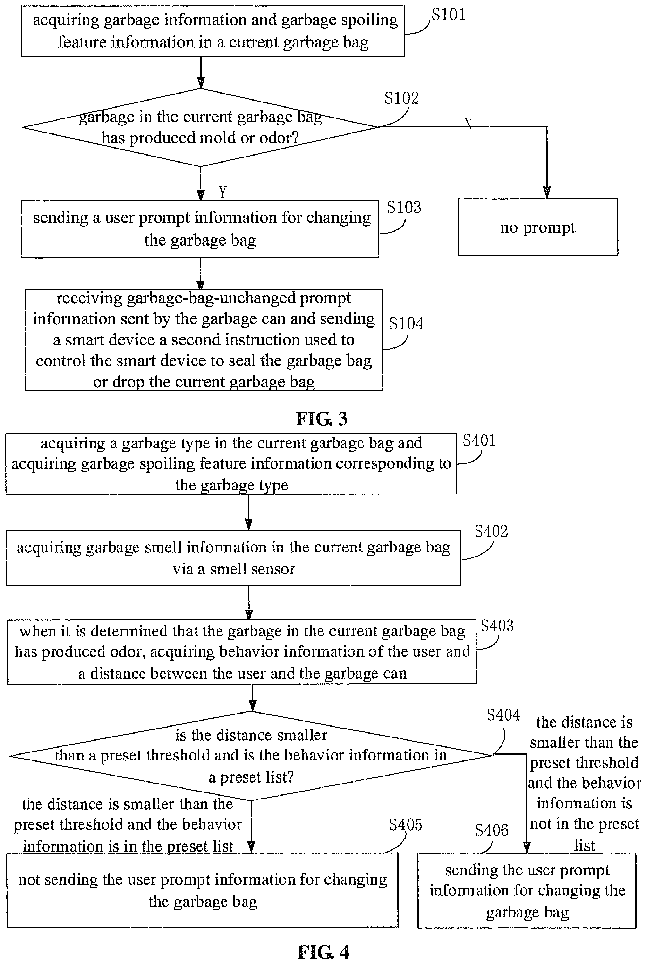

Fig. 3 is a flow chart showing another method for prompting change of garbage bag according to an exemplary embodiment. As shown in Fig. 3, based on the embodiment shown in Fig. 1, the present embodiment may further include the following steps.

In step S104, garbage-bag-unchanged prompt information sent by the garbage can may be received and a second instruction used to control a smart device to seal the garbage bag or drop the current garbage bag may be sent to the smart device.

In the present embodiment, after the mobile terminal such as a phone sends the user the prompt information for changing the garbage bag in various ways, if the garbage can detects that the weight of the garbage has not changed and still has mold or odor within a preset time period such as one hour, it can be confirmed that the user has not changed the garbage bag; then, the garbage can may send the mobile terminal garbage-bag-unchanged prompt information, upon receipt of which, the mobile terminal may send a second instruction to a smart device in the same local area network such as a robot; after receiving the second instruction, the smart device may seal the garbage bag or drop the current garbage bag.

With the above embodiment of the method for prompting change of garbage bag, by sending a smart device a second instruction to enable the smart device to seal the garbage bag or drop the current garbage bag after receiving garbage-bag-unchanged prompt information sent by the garbage can, a situation in which garbage pollutes the environment due to a user forgetting to change a garbage bag can be prevented effectively.

Fig. 4 is a flow chart showing a further method for prompting change of garbage bag according to an exemplary embodiment, which embodiment may be performed by a phone. As shown in Fig. 4, the method may include the following steps S401-S406.

In step S401, a garbage type in the current garbage bag may be acquired and garbage spoiling feature information corresponding to the garbage type may be acquired.

Implementation of the above step may be acquired by referring to the corresponding part of the embodiment shown in Fig. 1, which will not be described in detail herein.

In step S402, garbage smell information in the current garbage bag may be acquired via a smell sensor.

In step S403, when it is determined that the garbage in the current garbage bag has produced odor, behavior information of the user and a distance between the user and the garbage can may be acquired.

In step S404, whether the distance is smaller than a preset threshold and whether the behavior information is in a preset list may be determined.

The preset list is used to store information of a behavior that can produce odor, such as cooking behavior information.

In step S405, when it is determined that the distance is smaller than the preset threshold and the behavior information is in the preset list, prompt information for changing the garbage bag may not be sent to the user and operation ends.

For example, when cooking, a user will typically drop fish debris, etc. into a garbage can, and the garbage can will detect a fishy smell. However, since the distance between the user and the garbage can is less than a preset distance such as five meters and the cooking behavior information of the user is in a preset list, prompt information for changing the garbage bag will not be sent to the user. In such cases, the user typically needs to change the garbage bag after cooking.

In step S406, when it is determined that the distance is smaller than the preset threshold and the behavior information is not in the preset list, prompt information for changing the garbage bag may be sent to the user.

If the distance between the user and the garbage can is less than a preset distance such as five meters but the cooking behavior information of the user is not in the preset list (for example, the user is pouring water in the kitchen), prompt information for changing the garbage bag will be sent to the user.

In other cases, such as when the distance is larger than the preset distance, prompt information for changing the garbage bag will also be sent to the user.

With the above embodiment of the method for prompting change of garbage bag, when garbage smell information and garbage spoiling feature information in the current garbage bag are acquired and it is determined that the garbage in the current garbage bag has produced odor based on the garbage smell information and garbage spoiling feature information, by further acquiring behavior information of the user and a distance between the user and the garbage can and determining whether it is needed to send prompt information to a user based on this information to enable the user to change the garbage bag, situations in which prompt information is sent to a user due to misjudgments can be effectively reduced so that interruption to users can be reduced effectively; meanwhile, the impact on human health and the pollution of surrounding environment by garbage can be reduced effectively and use experience of the user can be improved.

Corresponding to above embodiments of methods for prompting change of garbage bag, the present invention further provides embodiments of devices for prompting change of garbage bag.

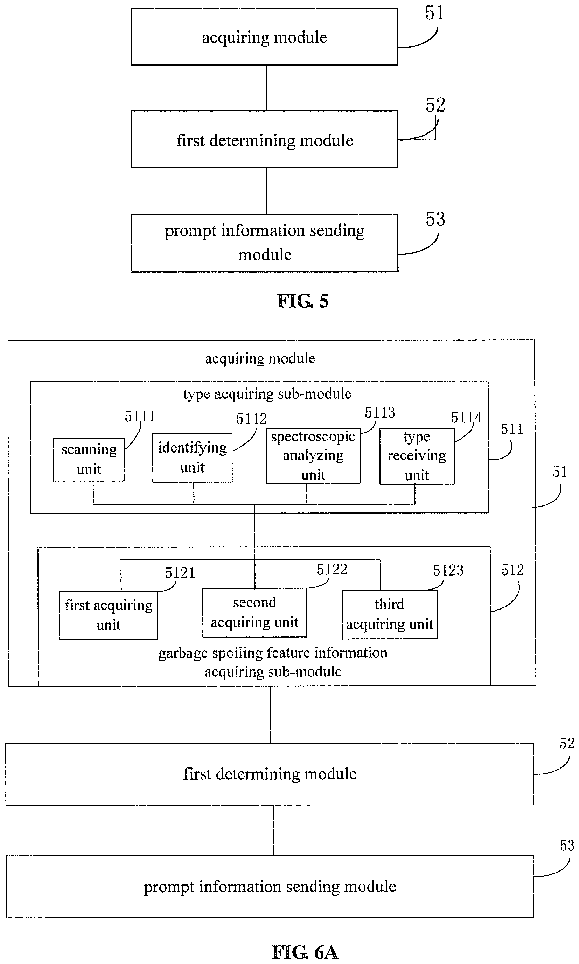

Fig. 5 is a block diagram showing a device for prompting change of garbage bag according to an exemplary embodiment. As shown in Fig. 5, the device for prompting change of garbage bag may include: an acquiring module 51, a first determining module 52 and a prompt information sending module 53.

The acquiring module 51 may be configured to acquire garbage information and garbage spoiling feature information in a current garbage bag.

The first determining module 52 may be configured to determine whether garbage in the current garbage bag has produced mold or odor based on the garbage information and the garbage spoiling feature information acquired by the acquiring module 51.

The first determining module 52 may be configured to determine whether the garbage information is consistent with at least a part of the garbage spoiling feature information, wherein the garbage spoiling feature information is used to determine whether the garbage in the current garbage bag has produced mold or odor.

The prompt information sending module 53 may be configured to, when the first determining module 52 determines that the garbage has produced mold or odor, send a user prompt information for changing the garbage bag.

The above embodiment of the device for prompting change of garbage bag acquires garbage information and garbage spoiling feature information in a current garbage bag by an acquiring module, determines whether garbage in the current garbage bag has produced mold or odor based on the garbage information and the garbage spoiling feature information by a first determining module, and sends a user prompt information for changing the garbage bag when it is determined that the garbage has produced mold or odor by a prompt information sending module to enable the user to change the garbage bag. Since the embodiment prompts the user to change the current garbage bag when it is determined that garbage has produced mold or odor, the impact on human health and the pollution of surrounding environment by garbage can be reduced effectively and use experience of the user can be improved.

Fig. 6A is a block diagram showing another device for prompting change of garbage bag according to an exemplary embodiment. As shown in Fig. 6A, based on the embodiment shown in Fig. 5, the acquiring module 51 may include: a type acquiring sub-module 511 and garbage spoiling feature information acquiring sub-module 512.

The type acquiring sub-module 511 may be configured to acquire a garbage type in the current garbage bag.

The garbage spoiling feature information acquiring sub-module 512 may be configured to acquire garbage spoiling feature information corresponding to the garbage type.

As shown in Fig. 6A, the type acquiring sub-module 511 may include: a scanning unit 5111, an identifying unit 5112, a spectroscopic analyzing unit 5113, or a type receiving unit 5114.

The scanning unit 5111 may be configured to acquire the garbage type by scanning a barcode on a packing box or on a packing bag of the garbage.

The identifying unit 5112 may be configured to acquire the garbage type by identifying the garbage via a camera.

The spectroscopic analyzing unit 5113 may be configured to acquire the garbage type by making a spectroscopic analysis on composition of the garbage.

The type receiving unit 5114 may be configured to receive the garbage type reported by the garbage can.

As shown in Fig. 6A, the garbage spoiling feature information acquiring sub-module 512 may include: a first acquiring unit 5121, a second acquiring unit 5122 or a third acquiring unit 5123.

The first acquiring unit 5121 may be configured to query a preset database based on the garbage type to acquire garbage spoiling feature information corresponding to the garbage type, the preset database storing spoiling feature information of different garbage types.

The second acquiring unit 5122 may be configured to acquire garbage spoiling feature information corresponding to the garbage type from prestored history record information.

The third acquiring unit 5123 may be configured to acquire garbage spoiling feature information corresponding to the garbage type based on user's review information.

The above embodiment of the device for prompting change of garbage bag provides various ways to acquire a garbage type and provides various ways to acquire garbage spoiling feature information based on the garbage type. As can be seen, the present embodiment may be implemented in flexible and diverse ways.

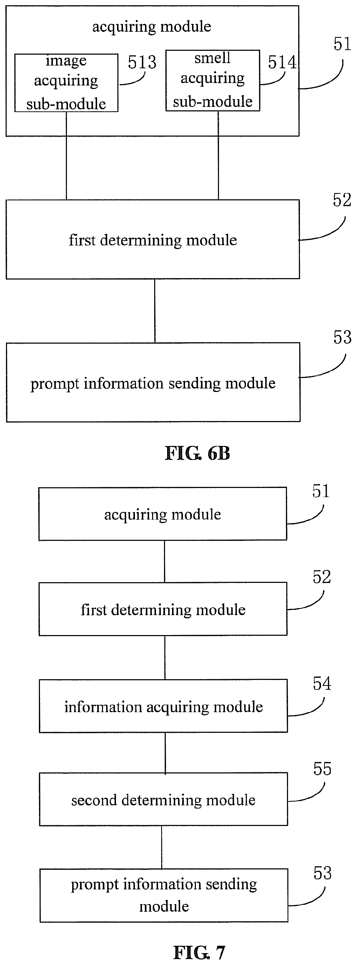

Fig. 6B is a block diagram showing another device for prompting change of garbage bag according to an exemplary embodiment. As shown in Fig. 6B, based on the embodiment shown in Fig. 5, the acquiring module 51 may include at least one of an image acquiring sub-module 513 and a smell acquiring sub-module 514.

The image acquiring sub-module 513 may be configured to acquire garbage image information in the current garbage bag via a camera.

The smell acquiring sub-module 514 may be configured to acquire garbage smell information in the current garbage bag via a smell sensor.

The above embodiment of the device for prompting change of garbage bag allows for subsequent operations by acquiring garbage information via a camera or a smell sensor.

Fig. 7 is a block diagram showing another device for prompting change of garbage bag according to an exemplary embodiment. As shown in Fig. 7, based on the embodiment shown in Fig. 5, the device may further include: an information acquiring module 54 and a second determining module 55.

The information acquiring module 54 may be configured to acquire behavior information of the user and a distance between the user and the garbage can, before the prompt information sending module 53 sends the user the prompt information for changing the garbage bag.

The second determining module 55 may be configured to determine whether the distance is smaller than a preset threshold and whether the behavior information is in a preset list, the preset list being used to store information of a behavior that can produce odor.

The above embodiment of the device for prompting change of garbage bag can effectively reduce situations in which prompt information is sent to a user due to misjudgments, and thus effectively reduces interruption to users and improves the degree of use experience of the user.

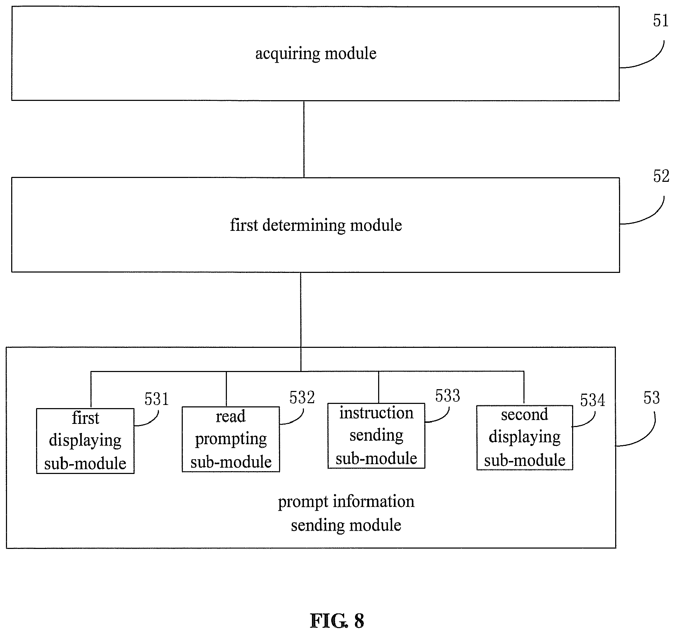

Fig. 8 is a block diagram showing another device for prompting change of garbage bag according to an exemplary embodiment. As shown in Fig. 8, based on the embodiment shown in Fig. 5, the prompt information sending module 53 may include one or more of the following sub-modules.

A first displaying sub-module 531 configured to display the prompt information for changing the garbage bag via a mobile terminal.

A read prompting sub-module 532 configured to prompt the user to read the prompt information for changing the garbage bag on the mobile terminal via a wearable device.

An instruction sending sub-module 533 configured to, when it is detected that the mobile terminal is in the same local area network as the garbage can, send the garbage can a first instruction, which is used to control the garbage can to make a sound to prompt the user to change the garbage bag.

A second displaying sub-module 534 configured to, when it is detected that the mobile terminal is in the same local area network as the garbage can, display the prompt information for changing the garbage bag via a terminal device which includes a TV.

The above embodiment of the device for prompting change of garbage bag can send a user prompt information for changing the garbage bag in various ways, and can be implemented in flexible and diverse ways.

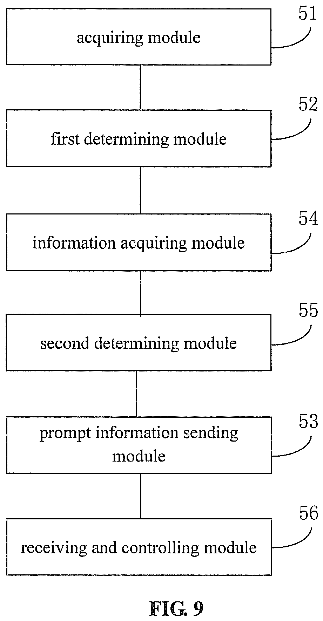

Fig. 9 is a block diagram showing another device for prompting change of garbage bag according to an exemplary embodiment. As shown in Fig. 9, based on the embodiment shown in Fig. 5, the device may further include: a receiving and controlling module 56.

The receiving and controlling module 56 may be configured to, after the prompt information sending module 53 sends the user the prompt information for changing the garbage bag, receive garbage-bag-unchanged prompt information sent by the garbage can and send a smart device a second instruction used to control the smart device to seal the garbage bag or drop the current garbage bag.

By sending a second instruction to a smart device after receiving garbage-bag-unchanged prompt information sent by the garbage can, the above embodiment of the device for prompting change of garbage bag enables the smart device to seal the garbage bag or drop the current garbage bag, and thus effectively reduces the impact on human health and the pollution of surrounding environment by garbage.

With respect to the devices in above embodiments, specific manners in which respective modules and sub-modules perform operations have been described in detail in embodiments related to methods, which will not be elaborated herein.

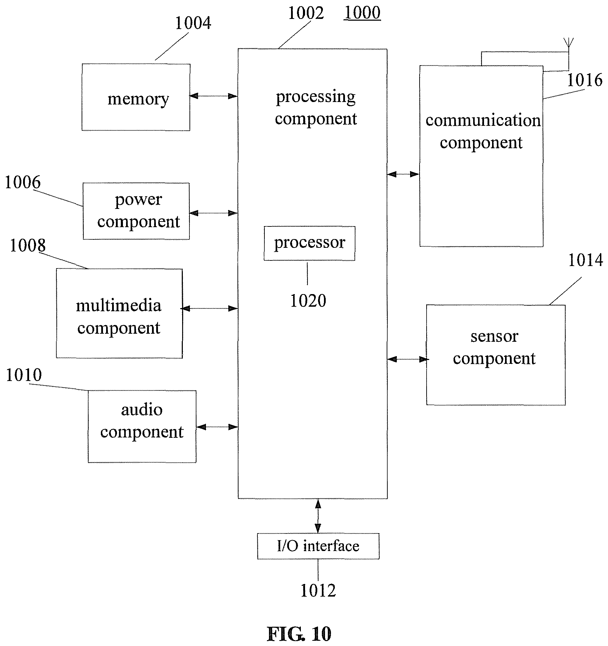

Fig. 10 is a block diagram applicable to a device for prompting change of garbage bag according to an exemplary embodiment. For example, device 1000 may be a mobile phone, a computer, a digital broadcast terminal, a messaging device, a gaming console, a tablet, a medical device, exercise equipment, a personal digital assistant, an aircraft and the like.

Referring to Fig. 10, the device 1000 may include one or more of the following components: a processing component 1002, a memory 1004, a power component 1006, a multimedia component 1008, an audio component 1010, an input/output (I/O) interface 1012, a sensor component 1014, and a communication component 1016.

The processing component 1002 typically controls overall operations of the device 1000, such as the operations associated with display, telephone calls, data communications, camera operations, and recording operations. The processing component 1002 may include one or more processors 1020 to execute instructions to perform all or part of the steps in the above described methods. Moreover, the processing component 1002 may include one or more modules which facilitate the interaction between the processing component 1002 and other components. For instance, the processing component 1002 may include a multimedia module to facilitate the interaction between the multimedia component 1008 and the processing component 1002.

The memory 1004 is configured to store various types of data to support the operation of the device 1000. Examples of such data include instructions for any applications or methods operated on the device 1000, contact data, phonebook data, messages, pictures, video, etc. The memory 1004 may be implemented using any type of volatile or non-volatile memory devices, or a combination thereof, such as a static random access memory (SRAM), an electrically erasable programmable read-only memory (EEPROM), an erasable programmable read-only memory (EPROM), a programmable read-only memory (PROM), a read-only memory (ROM), a magnetic memory, a flash memory, a magnetic or optical disk.

The power component 1006 provides power to various components of the device 1000. The power component 1006 may include a power management system, one or more power sources, and any other components associated with the generation, management, and distribution of power in the device 1000.

The multimedia component 1008 includes a screen providing an output interface between the device 1000 and the user. In some embodiments, the screen may include a liquid crystal display (LCD) and a touch panel (TP). If the screen includes the touch panel, the screen may be implemented as a touch screen to receive input signals from the user. The touch panel includes one or more touch sensors to sense touches, swipes, and gestures on the touch panel. The touch sensors may not only sense a boundary of a touch or swipe action, but also sense a period of time and a pressure associated with the touch or swipe action. In some embodiments, the multimedia component 1008 includes a front camera and/or a rear camera. The front camera and the rear camera may receive an external multimedia datum while the device 1000 is in an operation mode, such as a photographing mode or a video mode. Each of the front camera and the rear camera may be a fixed optical lens system or have focus and optical zoom capability.

The audio component 1010 is configured to output and/or input audio signals. For example, the audio component 1010 includes a microphone ("MIC") configured to receive an external audio signal when the device 1000 is in an operation mode, such as a call mode, a recording mode, and a voice recognition mode. The received audio signal may be further stored in the memory 1004 or transmitted via the communication component 1016. In some embodiments, the audio component 1010 further includes a speaker to output audio signals.

The I/O interface 1012 provides an interface between the processing component 1002 and peripheral interface modules, such as a keyboard, a click wheel, buttons, and the like. The buttons may include, but are not limited to, a home button, a volume button, a starting button, and a locking button.

The sensor component 1014 includes one or more sensors to provide status assessments of various aspects of the device 1000. For instance, the sensor component 1014 may detect an open/closed status of the device 1000, relative positioning of components, e.g., the display and the keypad, of the device 1000, a change in position of the device 1000 or a component of the device 1000, a presence or absence of user contact with the device 1000, an orientation or an acceleration/deceleration of the device 1000, and a change in temperature of the device 1000. The sensor component 1014 may include a proximity sensor configured to detect the presence of nearby objects without any physical contact. The sensor component 1014 may also include a light sensor, such as a CMOS or CCD image sensor, for use in imaging applications. In some embodiments, the sensor component 1014 may also include an accelerometer sensor, a gyroscope sensor, a magnetic sensor, a pressure sensor, or a temperature sensor.

The communication component 1016 is configured to facilitate communication, wired or wirelessly, between the device 1000 and other devices. The device 1000 can access a wireless network based on a communication standard, such as WiFi, 2G, or 3G, or a combination thereof. In one exemplary embodiment, the communication component 1016 receives a broadcast signal or broadcast associated information from an external broadcast management system via a broadcast channel. In one exemplary embodiment, the communication component 1016 further includes a near field communication (NFC) module to facilitate short-range communications. For example, the NFC module may be implemented based on a radio frequency identification (RFID) technology, an infrared data association (IrDA) technology, an ultra-wideband (UWB) technology, a Bluetooth (BT) technology, and other technologies.

In exemplary embodiments, the device 1000 may be implemented with one or more application specific integrated circuits (ASICs), digital signal processors (DSPs), digital signal processing devices (DSPDs), programmable logic devices (PLDs), field programmable gate arrays (FPGAs), controllers, micro-controllers, microprocessors, or other electronic components, for performing the above described methods.

In exemplary embodiments, there is also provided a non-transitory computer-readable storage medium including instructions, such as included in the memory 1004, executable by the processor 1020 in the device 1000, for performing the above-described methods. For example, the non-transitory computer-readable storage medium may be a ROM, a RAM, a CD-ROM, a magnetic tape, a floppy disc, an optical data storage device, and the like.

Other embodiments of the invention will be apparent to those skilled in the art from consideration of the specification and practice of the invention disclosed here. This application is intended to cover any variations, uses, or adaptations of the invention following the general principles thereof and including such departures from the present invention as come within known or customary practice in the art. The specification and embodiments are merely considered to be exemplary and the substantive scope of the invention is limited only by the appended claims.

It should be understood that the invention is not limited to the precise structure as described above and shown in the figures, but can have various modification and alternations without departing from the scope of the invention. The scope of the invention is limited only by the appended claims.

## Claims

1. A method for prompting the change of a garbage bag, comprising:
acquiring (S101) garbage information and garbage spoiling feature information in a current garbage bag;
determining (S102) whether garbage in the current garbage bag has produced mold or odor based on the garbage information and the garbage spoiling feature information; and
when it is determined that the garbage has produced mold or odor, sending (S103) a user prompt information for changing the garbage bag, **characterized in that** the step (S101) of acquiring garbage spoiling feature information includes:
acquiring a garbage type in the current garbage bag; and
acquiring garbage spoiling feature information corresponding to the garbage type;
and **in that** the method for prompting the change of a garbage bag is performed by a mobile terminal.

2. The method of claim 1, wherein the step of acquiring a garbage type in the current garbage bag includes:
acquiring the garbage type by scanning a barcode on a packing box or on a packing bag of the garbage; or
acquiring the garbage type by identifying the garbage via a camera; or
acquiring the garbage type by making a spectroscopic analysis on composition of the garbage; or
receiving the garbage type reported by a garbage can.

3. The method of claim 1, wherein the step of acquiring garbage spoiling feature information corresponding to the garbage type includes:
querying a preset database based on the garbage type to acquire garbage spoiling feature information corresponding to the garbage type, the preset database storing spoiling feature information of different garbage types; or
acquiring garbage spoiling feature information corresponding to the garbage type from prestored history record information; or
acquiring garbage spoiling feature information corresponding to the garbage type based on user's review information.

4. The method of claim 1, wherein the step (S101) of acquiring garbage information in the current garbage bag includes:
acquiring garbage image information in the current garbage bag via a camera; and/or
acquiring garbage smell information in the current garbage bag via a smell sensor.

5. The method of claim 1, wherein the step (S102) of determining whether garbage in the current garbage bag has produced mold or odor based on the garbage information and the garbage spoiling feature information includes:
determining whether the garbage information is consistent with at least a part of the garbage spoiling feature information, the garbage spoiling feature information being used to determine whether the garbage in the current garbage bag has produced mold or odor.

6. The method of claim 1, wherein the method further includes:
before sending the user the prompt information for changing the garbage bag, acquiring (S403) behavior information of the user and a distance between the user and a garbage can; and
determining (S404) whether the distance is smaller than a preset threshold and whether the behavior information is in a preset list, the preset list being used to store information of a behavior that can produce odor.

7. The method of claim 6, wherein the step (S103) of sending a user prompt information for changing the garbage bag includes:
when it is determined that the distance is smaller than the preset threshold and the behavior information is not in the preset list, sending (S406) the user the prompt information for changing the garbage bag.

8. The method of claim 1, wherein the step (S103) of sending a user prompt information for changing the garbage bag includes:
displaying the prompt information for changing the garbage bag via a mobile terminal; and/or
prompting the user to read the prompt information for changing the garbage bag on the mobile terminal via a wearable device; and/or
when it is detected that the mobile terminal is in the same local area network as a garbage can, sending the garbage can a first instruction, which is used to control the garbage can to make a sound to prompt the user to change the garbage bag; and/or
when it is detected that the mobile terminal is in the same local area network as the garbage can, displaying the prompt information for changing the garbage bag via a terminal device which includes a TV.

9. The method of claim 1, wherein the method further includes:
after sending the user the prompt information for changing the garbage bag, receiving (S104) garbage-bag-unchanged prompt information sent by a garbage can and sending a smart device a second instruction used to control the smart device to seal the garbage bag or drop the current garbage bag.

10. A device for prompting the change of a garbage bag comprising:
an acquiring module (51) configured to acquire garbage information and garbage spoiling feature information in a current garbage bag;
a first determining module (52) configured to determine whether garbage in the current garbage bag has produced mold or odor based on the garbage information and the garbage spoiling feature information acquired by the acquiring module (51); and
a prompt information sending module (53) configured to, when the first determining module (52) determines that the garbage has produced mold or odor, send a user prompt information for changing the garbage bag,
**characterized in that** wherein the acquiring module (51) includes:
a type acquiring sub-module (511) configured to acquire a garbage type in the current garbage bag; and
a garbage spoiling feature information acquiring sub-module (512) configured to acquire garbage spoiling feature information corresponding to the garbage type;
and **in that** the device for prompting the change of a garbage bad is in a mobile terminal.

11. The device of claim 10, wherein the garbage spoiling feature information acquiring sub-module (512) includes:
a first acquiring unit (5121) configured to query a preset database based on the garbage type to acquire garbage spoiling feature information corresponding to the garbage type, the preset database storing spoiling feature information of different garbage types; or
a second acquiring unit (5122) configured to acquire garbage spoiling feature information corresponding to the garbage type from prestored history record information; or
a third acquiring unit (5123) configured to acquire garbage spoiling feature information corresponding to the garbage type based on user's review information.

12. The device of claim 10, wherein the acquiring module (51) includes:
an image acquiring sub-module (513) configured to acquire garbage image information in the current garbage bag via a camera; and/or
a smell acquiring sub-module (514) configured to acquire garbage smell information in the current garbage bag via a smell sensor.

13. The device of claim 10, wherein the first determining module (52) is configured to:
determine whether the garbage information is consistent with at least a part of the garbage spoiling feature information, the garbage spoiling feature information being used to determine whether the garbage in the current garbage bag has produced mold or odor.

14. The device of claim 10, wherein the device further includes:
an information acquiring module (54) configured to, before the prompt information sending module (53) sends the user the prompt information for changing the garbage bag, acquire behavior information of the user and a distance between the user and a garbage can; and
a second determining module (55) configured to determine whether the distance is smaller than a preset threshold and whether the behavior information is in a preset list, the preset list being used to store information of a behavior that can produce odor,
wherein the prompt information sending module (53) is configured to:
when it is determined that the distance is smaller than the preset threshold and the behavior information is not in the preset list, send the user the prompt information for changing the garbage bag.

## Patentansprüche

1. Verfahren zum Auffordern, einen Müllbeutel zu tauschen, umfassend:
Erfassen (S101) von Abfallinformationen und Abfallverderbmerkmalsinformationen in einem vorliegenden Müllbeutel,
Bestimmen (S102), ob Abfall in dem vorliegenden Müllbeutel Schimmel oder Gerüche produziert hat, basierend auf den Abfallinformationen und den Abfallverderbmerkmalsinformationen, und
wenn bestimmt wird, dass der Abfall Schimmel oder Gerüche produziert hat, Senden (S103) von Aufforderungsinformationen an einen Benutzer, den Müllbeutel zu tauschen,
**dadurch gekennzeichnet, dass**
der Schritt (S101) des Erfassens von Abfallverderbmerkmalsinformationen beinhaltet:
Erfassen einer Abfallart in dem vorliegenden Müllbeutel und
Erfassen von Abfallverderbmerkmalsinformationen entsprechend der Abfallart,
und dadurch, dass das Verfahren zum Auffordern, einen Müllbeutel zu tauschen, durch ein mobiles Endgerät durchgeführt wird.

2. Verfahren nach Anspruch 1, wobei der Schritt des Erfassens einer Abfallart in dem vorliegenden Müllbeutel beinhaltet:
Erfassen der Abfallart durch Scannen eines Barcodes auf einer Verpackungsbox oder einem Verpackungsbeutel des Abfalls oder
Erfassen der Abfallart durch Identifizieren des Abfalls über eine Kamera oder
Erfassen der Abfallart durch Vornehmen einer spektroskopischen Analyse der Zusammensetzung des Abfalls, oder
Empfangen der von einem Abfalleimer gemeldeten Abfallart.

3. Verfahren nach Anspruch 1, wobei der Schritt des Erfassens von Abfallverderbmerkmalsinformationen entsprechend der Abfallart beinhaltet:
Abfragen einer voreingestellten Datenbank basierend auf der Abfallart, um Abfallverderbmerkmalsinformationen entsprechend der Abfallart zu erfassen, wobei die voreingestellte Datenbank Abfallverderbmerkmalsinformationen unterschiedlicher Abfallarten speichert, oder
Erfassen von Abfallverderbmerkmalsinformationen entsprechend der Abfallart aus vorgespeicherten Verlaufaufzeichnungsinformationen, oder
Erfassen von Abfallverderbmerkmalsinformationen entsprechend der Abfallart basierend auf Bewertungsinformationen eines Benutzers.

4. Verfahren nach Anspruch 1, wobei der Schritt (S101) des Erfassens von Abfallinformationen in dem vorliegenden Müllbeutel beinhaltet:
Erfassen von Abfallbildinformationen in dem vorliegenden Müllbeutel über eine Kamera und/oder
Erfassen von Abfallgeruchsinformationen in dem vorliegenden Müllbeutel über einen Geruchssensor.

5. Verfahren nach Anspruch 1 wobei der Schritt (S102) des Bestimmens, ob Abfall in dem vorliegenden Müllbeutel Schimmel oder Gerüche produziert hat, basierend auf den Abfallinformationen und den Abfallverderbmerkmalsinformationen beinhaltet:
Bestimmen, ob die Abfallinformationen mit mindestens einem Teil der Abfallverderbmerkmalsinformationen übereinstimmen, wobei die Abfallverderbmerkmalsinformationen verwendet werden, um zu bestimmen, ob der Abfall in dem vorliegenden Müllbeutel Schimmel oder Gerüche produziert hat.

6. Verfahren nach Anspruch 1, wobei das Verfahren ferner beinhaltet:
Erfassen (S403), bevor dem Benutzer die Aufforderungsinformationen zum Austauschen des Müllbeutels gesendet werden, von Verhaltensinformationen des Benutzers und eines Abstandes zwischen dem Benutzer und einem Abfalleimer, und
Bestimmen (S404), ob der Abstand kleiner als ein voreingestellter Schwellenwert ist, und ob sich die Verhaltensinformationen in einer voreingestellten Liste befinden, wobei die voreingestellte Liste verwendet wird, um Informationen über ein Verhalten, das Gerüche produzieren kann, zu speichern.

7. Verfahren nach Anspruch 6, wobei der Schritt (S103) des Sendens von Aufforderungsinformationen an den Benutzer, den Müllbeutel zu tauschen, beinhaltet:
Senden (S406), wenn bestimmt wird, dass der Abstand kleiner als ein voreingestellter Schwellenwert ist und sich die Verhaltensinformationen nicht in der voreingestellten Liste befinden, der Aufforderungsinformationen zum Austauschen des Müllbeutels an den Benutzer.

8. Verfahren nach Anspruch 1, wobei der Schritt (S103) des Sendens von Aufforderungsinformationen zum Austauschen des Müllbeutels an den Benutzer beinhaltet:
Anzeigen der Aufforderungsinformationen, den Müllbeutel zu tauschen, über ein mobiles Endgerät und/oder
Auffordern des Benutzers mittels einer tragbaren Vorrichtung, die Aufforderungsinformationen zum Austauschen des Müllbeutels auf dem mobilen Endgerät zu lesen, und/oder
wenn erkannt wird, dass sich das mobile Endgerät in demselben lokalen Netzwerk wie der Abfalleimer befindet, Senden einer ersten Anweisung an den Abfalleimer, die verwendet wird, um den Abfalleimer so zu steuern, dass er einen Ton ausgibt, um den Benutzer aufzufordern, den Müllbeutel auszutauschen, und/oder
wenn erkannt wird, dass sich das mobile Endgerät in demselben lokalen Netzwerk wie der Abfalleimer befindet, Anzeigen der Aufforderungsinformationen, den Müllbeutel zu tauschen, über ein Endgerät, das einen Fernseher beinhaltet.

9. Verfahren nach Anspruch 1, wobei das Verfahren ferner beinhaltet:
Empfangen (S104), nach Senden von Aufforderungsinformationen, den Müllbeutel zu tauschen, an den Benutzer, von Müllbeutel-nicht-ausgetauscht-Aufforderungsinformationen, die von einem Abfalleimer gesendet werden, und Senden einer zweiten Anweisung an eine intelligente Vorrichtung, die verwendet wird, um die intelligente Vorrichtung zu steuern, um den Müllbeutel zu verschließen oder den vorliegenden Müllbeutel abzusetzen.

10. Vorrichtung zum Auffordern, einen Müllbeutel zu tauschen, umfassend:
ein Erfassungsmodul (51), das konfiguriert ist, Abfallinformationen und Abfallverderbmerkmalsinformationen in einem vorliegenden Müllbeutel zu erfassen,
ein erstes Bestimmungsmodul (52), das konfiguriert ist, basierend auf den Abfallinformationen und den Abfallverderbmerkmalsinformationen, die durch das Erfassungsmodul (51) erfasst werden, zu bestimmen, ob Abfall in dem vorliegenden Müllbeutel Schimmel oder Gerüche produziert hat, und
ein Aufforderungsinformationen-Sendemodul (53), das konfiguriert ist, einem Benutzer Aufforderungsinformationen, einen Müllbeutel zu tauschen, zu senden, wenn das erste Bestimmungsmodul (52) bestimmt, dass der Müll Schimmel oder Gerüche produziert hat,
**dadurch gekennzeichnet, dass** das Erfassungsmodul (51) beinhaltet:
ein Arterfassungsuntermodul (511), das konfiguriert ist, eine Abfallart in dem vorliegenden Müllbeutel zu erfassen, und
ein Abfallverderbmerkmalsinformationen-Erfassungsuntermodul (512), das konfiguriert ist, Abfallverderbmerkmalsinformationen entsprechend der Abfallart zu erfassen,
und dadurch, dass sich die Vorrichtung zur Aufforderung des Austauschs eines Müllbeutels in einem mobilen Endgerät befindet.

11. Vorrichtung nach Anspruch 10, wobei das Abfallverderbmerkmalsinformationen-Erfassungsuntermodul (512) beinhaltet:
eine erste Erfassungseinheit (5121), die konfiguriert ist, eine voreingestellte Datenbank basierend auf der Abfallart abzufragen, um Abfallverderbmerkmalsinformationen entsprechend der Abfallart zu erfassen, wobei die voreingestellte Datenbank Abfallverderbmerkmalsinformationen unterschiedlicher Abfallarten speichert, oder
eine zweite Erfassungseinheit (5122), die konfiguriert ist, Abfallverderbmerkmalsinformationen entsprechend der Abfallart aus vorgespeicherten Verlaufaufzeichnungsinformationen zu erfassen, oder
eine dritte Erfassungseinheit (5123), die konfiguriert ist, Abfallverderbmerkmalsinformationen entsprechend der Abfallart basierend auf Bewertungsinformationen eines Benutzers zu erfassen.

12. Vorrichtung nach Anspruch 10, wobei das Erfassungsmodul (51) beinhaltet:
ein Bilderfassungsuntermodul (513), das konfiguriert ist, Abfallbildinformationen in dem vorliegenden Müllbeutel über eine Kamera zu erfassen, und/oder
ein Geruchserfassungsuntermodul (514), das konfiguriert ist, Abfallgeruchsinformationen in dem vorliegenden Müllbeutel über einen Geruchssensor zu erfassen.

13. Vorrichtung nach Anspruch 10, wobei das erste Bestimmungsmodul (52) konfiguriert ist:
zu bestimmen, ob die Abfallinformationen mit mindestens einem Teil der Abfallverderbmerkmalsinformationen übereinstimmen, wobei die Abfallverderbmerkmalsinformationen verwendet werden, um zu bestimmen, ob der Abfall in dem vorliegenden Müllbeutel Schimmel oder Gerüche produziert hat.

14. Vorrichtung nach Anspruch 10, wobei die Vorrichtung ferner beinhaltet:
ein Informationserfassungsmodul (54), das konfiguriert ist, bevor das Aufforderungsinformationen-Sendemodul (53) dem Benutzer die Aufforderungsinformationen, den Müllbeutel zu tauschen, sendet, Verhaltensinformationen des Benutzers und einen Abstand zwischen dem Benutzer und einem Abfalleimer zu erfassen, und
ein zweites Bestimmungsmodul (55), das konfiguriert ist, zu bestimmen, ob der Abstand kleiner als ein voreingestellter Schwellenwert ist, und ob sich die Verhaltensinformationen in einer voreingestellten Liste befinden, wobei die voreingestellte Liste verwendet wird, um Informationen über ein Verhalten, das Gerüche produzieren kann, zu speichern, wobei das Aufforderungsinformationen-Sendemodul (53) konfiguriert ist:
dem Benutzer, wenn bestimmt wird, dass der Abstand kleiner als ein voreingestellter Schwellenwert ist und sich die Verhaltensinformationen nicht in der Voreinstellungsliste befinden, die Aufforderungsinformationen, den Müllbeutel zu tauschen, zu senden.

## Revendications

1. Procédé pour inviter au changement d'un sac à ordures, comprenant :
l'acquisition (S101) d'informations d'ordures et d'informations de caractéristique de détérioration d'ordures dans un sac à ordures actuel ;
la détermination (S102) de si des ordures dans le sac à ordures actuel ont produit de la moisissure ou une mauvaise odeur sur la base des informations d'ordures et des informations de caractéristique de détérioration d'ordures : et
lorsqu'il est déterminé que les ordures ont produit de la moisissure ou une mauvaise odeur, l'envoi (S103) d'une information d'invite d'utilisateur pour changer le sac à ordures,
**caractérisé en ce que**
l'étape (S101) d'acquisition d'informations de caractéristique de détérioration d'ordures comprend :
l'acquisition d'un type d'ordures dans le sac à ordures actuel ; et
l'acquisition d'informations de caractéristique de détérioration d'ordures correspondant au type d'ordures ;
et **en ce que** le procédé pour inviter au changement d'un sac à ordures est effectué par un terminal mobile.

2. Procédé selon la revendication 1, dans lequel l'étape d'acquisition d'un type d'ordures dans le sac à ordures courant comprend :
l'acquisition du type d'ordures en scannant un code à barres sur une boîte d'emballage ou sur un sac d'emballage des ordures ; ou
l'acquisition du type d'ordures en identifiant les ordures via une caméra ; ou
l'acquisition du type d'ordures en faisant une analyse spectroscopique sur la composition des ordures ; ou
la réception du type d'ordures rapporté par une poubelle.

3. Procédé selon la revendication 1, dans lequel l'étape d'acquisition d'informations de caractéristique de détérioration d'ordures correspondant au type d'ordures comprend :
l'interrogation d'une base de données prédéfinie sur la base du type d'ordures pour acquérir des informations de caractéristique de détérioration d'ordures correspondant au type d'ordures, la base de données prédéfinie stockant des informations de caractéristique de détérioration de différents types d'ordures ; ou
l'acquisition d'informations de caractéristique de détérioration d'ordures correspondant au type d'ordures à partir d'informations d'enregistrement d'historique préstockées ; ou
l'acquisition d'informations de caractéristique de détérioration d'ordures correspondant au type d'ordures sur la base d'informations d'examen de l'utilisateur.

4. Procédé selon la revendication 1, dans lequel l'étape (S101) d'acquisition d'informations d'ordures dans le sac à ordures actuel comprend :
l'acquisition d'informations d'image d'ordures dans le sac à ordures actuel via une caméra ; et/ou
l'acquisition d'informations d'odeur d'ordures dans le sac à ordures actuel via un capteur d'odeur.

5. Procédé selon la revendication 1, dans lequel l'étape (S102) de détermination de si des ordures dans le sac à ordures actuel ont produit de la moisissure ou une mauvaise odeur sur la base des informations d'ordures et des informations de caractéristique de détérioration d'ordures comprend :
la détermination de si les informations d'ordures sont compatibles avec au moins une partie des informations de caractéristique de détérioration d'ordures, les informations de caractéristique de détérioration d'ordures étant utilisées pour déterminer si les ordures dans le sac à ordures actuel ont produit de la moisissure ou une mauvaise odeur.

6. Procédé selon la revendication 1, dans lequel le procédé comprend en outre :
avant l'envoi à l'utilisateur de l'information d'invite pour changer le sac à ordures, l'acquisition (S403) d'informations de comportement de l'utilisateur et d'une distance entre l'utilisateur et une poubelle ; et
la détermination (S404) de si la distance est inférieure à un seuil prédéfini et de si les informations de comportement sont dans une liste prédéfinie, la liste prédéfinie étant utilisée pour stocker des informations d'un comportement qui peut produire une mauvaise odeur.

7. Procédé selon la revendication 6, dans lequel l'étape (S103) d'envoi d'une information d'invite d'utilisateur pour changer le sac à ordures comprend :
lorsqu'il est déterminé que la distance est inférieure au seuil prédéfini et que les informations de comportement ne sont pas dans la liste prédéfinie, l'envoi (S406) à l'utilisateur de l'information d'invite pour changer le sac à ordures.

8. Procédé selon la revendication 1, dans lequel l'étape (S103) d'envoi d'une information d'invite d'utilisateur pour changer le sac à ordures comprend :
l'affichage de l'information d'invite pour changer le sac à ordures via un terminal mobile ; et/ou
l'invitation de l'utilisateur à lire l'information d'invite pour changer le sac à ordures sur le terminal mobile via un dispositif portable ; et/ou
lorsqu'il est détecté que le terminal mobile se trouve dans le même réseau local qu'une poubelle, l'envoi à la poubelle d'une première instruction, qui est utilisée pour commander la poubelle pour émettre un son pour inviter l'utilisateur à changer le sac à ordures ; et/ou
lorsqu'il est détecté que le terminal mobile se trouve dans le même réseau local que la poubelle, l'affichage de l'information d'invite pour changer le sac à ordures via un dispositif terminal qui comprend une TV.

9. Procédé selon la revendication 1, dans lequel le procédé comprend en outre :
après l'envoi à l'utilisateur de l'information d'invite pour changer le sac à ordures, la réception (S104) d'une information d'invite sac à ordures non changé envoyée par une poubelle et l'envoi à un dispositif intelligent d'une seconde instruction utilisée pour commander le dispositif intelligent pour fermer le sac à ordures ou jeter le sac à ordures actuel.

10. Dispositif pour inviter au changement d'un sac à ordures comprenant :
un module d'acquisition (51) configuré pour acquérir des informations d'ordures et des informations de caractéristique de détérioration d'ordures dans un sac à ordures actuel ;
un premier module de détermination (52) configuré pour déterminer si des ordures dans le sac à ordures actuel ont produit de la moisissure ou une mauvaise odeur sur la base des informations d'ordures et des informations de caractéristique de détérioration d'ordures acquises par le module d'acquisition (51) ; et
un module d'envoi d'information d'invite (53) configuré pour, lorsque le premier module de détermination (52) détermine que les ordures ont produit de la moisissure ou une mauvaise odeur, envoyer une information d'invite d'utilisateur pour changer le sac à ordures,
**caractérisé en ce que** le module d'acquisition (51) comprend :
un sous-module d'acquisition de type (511) configuré pour acquérir un type d'ordures dans le sac à ordures actuel ; et
un sous-module d'acquisition d'informations de caractéristique de détérioration d'ordures (512) configuré pour acquérir des informations de caractéristique de détérioration d'ordures correspondant au type d'ordures ;
et **en ce que** le dispositif pour inviter au changement d'un sac à ordures se trouve dans un terminal mobile.

11. Dispositif selon la revendication 10, dans lequel le sous-module d'acquisition d'informations de caractéristique de détérioration d'ordures (512) comprend :
une première unité d'acquisition (5121) configurée pour interroger une base de données prédéfinie sur la base du type d'ordures pour acquérir des informations de caractéristique de détérioration d'ordures correspondant au type d'ordures, la base de données prédéfinie stockant des informations de caractéristique de détérioration de différents types d'ordures ; ou
une deuxième unité d'acquisition (5122) configurée pour acquérir des informations de caractéristique de détérioration d'ordures correspondant au type d'ordures à partir d'informations d'enregistrement d'historique préstockées ; ou
une troisième unité d'acquisition (5123) configurée pour acquérir des informations de caractéristique de détérioration d'ordures correspondant au type d'ordures sur la base d'informations d'examen de l'utilisateur.

12. Dispositif selon la revendication 10, dans lequel le module d'acquisition (51) comprend :
un sous-module d'acquisition d'image (513) configuré pour acquérir des informations d'image d'ordures dans le sac à ordures actuel via une caméra ; et/ou
un sous-module d'acquisition d'odeur (514) configuré pour acquérir des informations d'odeur d'ordures dans le sac à ordures actuel via un capteur d'odeur.

13. Dispositif selon la revendication 10, dans lequel le premier module de détermination (52) est configuré pour :
déterminer si les informations d'ordures sont compatibles avec au moins une partie des informations de caractéristique de détérioration d'ordures, les informations de caractéristique de détérioration d'ordures étant utilisées pour déterminer si les ordures dans le sac à ordures actuel ont produit de la moisissure ou une mauvaise odeur.

14. Dispositif selon la revendication 10, dans lequel le dispositif comprend en outre :
un module d'acquisition d'informations (54) configuré pour, avant que le module d'envoi d'information d'invite (53) n'envoie à l'utilisateur l'information d'invite pour changer le sac à ordures, acquérir des informations de comportement de l'utilisateur et une distance entre l'utilisateur et une poubelle ; et
un second module de détermination (55) configuré pour déterminer si la distance est inférieure à un seuil prédéfini et si les informations de comportement sont dans une liste prédéfinie, la liste prédéfinie étant utilisée pour stocker des informations d'un comportement qui peut produire une mauvaise odeur, dans lequel le module d'envoi d'information d'invite (53) est configuré pour :
lorsqu'il est déterminé que la distance est inférieure au seuil prédéfini et que les informations de comportement ne sont pas dans la liste prédéfinie, envoyer à l'utilisateur l'information d'invite pour changer le sac à ordures.
